# EUROPEAN PATENT APPLICATION

(11) **EP 3 527 226 A1**
(43) Date of publication of application: **21.08.2019**
(21) Application number: 17861727.0
(22) Date of filing: 16.10.2017
(51) Int. Cl.: A61K 45/00, A61K 31/4439, A61K 31/706, A61P 35/00, A61P 35/02, A61P 43/00

(54) **COMBINATION THERAPY METHOD USING MDM2 INHIBITOR AND DNA METHYLTRANSFERASE INHIBITOR**

(30) Priority: 17.10.2016 JP 2016203718
(71) Applicant: Daiichi Sankyo Company, Limited, Tokyo 103-8426 (JP)
(72) Inventor: SEKI, Takahiko, Tokyo 103-8426 (JP)
(74) Representative: Wallace, Sheila Jane
(86) International application number: PCT/JP2017/037284
(87) International publication number: WO 2018/074387

(57) **Abstract**

It is intended to provide a medicament and a method for treating cancer, comprising a compound having MDM2 inhibiting activity and a DNA methyltransferase inhibitor in combination. The present invention provides a medicament comprising (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor in combination, and a treatment method using the compound or a salt thereof and a DNA methyltransferase inhibitor in combination.

## Description

### Technical Field

The present invention relates to a medicament and a method for treating cancer comprising a compound having murine double minute 2 (MDM2) inhibiting activity and a DNA methyltransferase inhibitor in combination.

### Background Art

p53 is known as an important factor for inhibiting canceration of cells. p53 is a transcription factor that induces the expression of genes involved in the cell cycle and cellular apoptosis in response to various stresses. p53 is thought to inhibit canceration of cells by a transcription regulating function thereof. In fact, deletion or mutation of the p53 gene is observed in about half of human cancer cases.

Meanwhile, overexpression of murine double minute 2 (MDM2), a type of E3 ubiquitin ligase, is known as a factor for canceration. MDM2 is a protein whose expression is induced by p53. MDM2 negatively regulates p53 by binding to the transcription activity domain of p53 to decrease the transcription activity of p53, exporting p53 out of the nucleus, and further mediating degradation of p53 by acting as an ubiquitination ligase against p53. Therefore, it is thought that inactivation of functions of and degradation of p53 are promoted in cells in which MDM2 is overexpressed, resulting in canceration (Non Patent Document 1).

Paying attention to such functions of MDM2, many approaches have been attempted using substances that inhibit the suppression of p53 functions by MDM2 as candidate anti-tumor agents. Examples of MDM2 inhibitors targeting the MDM2-p53 binding site have been reported, which include spirooxindole derivatives (Patent Documents 1 to 15 and Non Patent Documents 1 to 3), indole derivatives (Patent Document 16), pyrrolidine-2-carboxamide derivatives (Patent Document 17), pyrrolidinone derivatives (Patent Document 18), isoindolinone derivatives (Patent Document 19 and Non Patent Document 4) and dispiropyrrolidine compounds (Patent Document 20).

In recent years, epigenomic abnormality has received attention as a new mechanism of canceration. It has been revealed that in addition to DNA sequence abnormality, epigenomic abnormality such as DNA hypermethylation or histone hypermodification is deeply involved in inactivation of a tumor suppressor gene or abnormal activation of an oncogene. Pharmaceutical products (epigenome drugs) are under development focusing on the epigenomic abnormality (Non Patent Documents 5 to 7).

DNA methyltransferase is an enzyme that catalyzes the methylation of DNA. Abnormal activation of DNA methyltransferase in various tumors has been reported, and DNA methyltransferase is one of the most known targets of epigenome drugs. Nucleoside analogs such as azacitidine and decitabine have been known as synthetic nucleic acid inhibitors from a long time ago. These drugs, however, had later been found to restore the highly methylated state to its normal state by inhibiting DNA methyltransferase. Therefore, azacitidine in 2004 and decitabine in 2006 were approved as epigenome drugs by the FDA in the USA.

DNA methyltransferase inhibitors such as azacitidine and decitabine have also been increasingly studied for combined use with other anti-cancer agents (Non Patent Document 5).

### Citation List

### Patent Document

Patent Document 1: WO2006/091646
Patent Document 2: WO2006/136606
Patent Document 3: WO2007/104664
Patent Document 4: WO2007/104714
Patent Document 5: WO2008/034736
Patent Document 6: WO2008/036168
Patent Document 7: WO2008/055812
Patent Document 8: WO2008/141917
Patent Document 9: WO2008/141975
Patent Document 10: WO2009/077357
Patent Document 11: WO2009/080488
Patent Document 12: WO2010/084097
Patent Document 13: WO2010/091979
Patent Document 14: WO2010/094622
Patent Document 15: WO2010/121995
Patent Document 16: WO2008/119741
Patent Document 17: WO2010/031713
Patent Document 18: WO2010/028862
Patent Document 19: WO2006/024837
Patent Document 20: WO2012/121361
Patent Document 21: WO2008/039218
Patent Document 22: WO2013/059738

### Non Patent Document

Non Patent Document 1: J. Am. Chem. Soc., 2005, 127, 10130-10131
Non Patent Document 2: J. Med. Chem., 2006, 49, 3432-3435
Non Patent Document 3: J. Med. Chem., 2009, 52, 7970-7973
Non Patent Document 4: J. Med. Chem., 2006, 49, 6209-6221
Non Patent Document 5: Trends Pharmacol. Sci., 2010, 31, 536-546
Non Patent Document 6: Drug Discov. Today, 2011, 16, 418-425
Non Patent Document 7: Cell. Signal., 2011, 23, 1082-1093

### Summary of Invention

### Technical Problem

The present invention provides a medicament and a method for treating cancer comprising a compound having MDM2 inhibiting activity and a DNA methyltransferase inhibitor in combination. The present invention provides an excellent medicament and method for treating cancer with a significantly high anti-tumor effect.

### Solution to the Problem

As a result of extensive studies, the present inventors have found that use of (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide, which is a compound having MDM2 inhibiting activity, or a pharmaceutically acceptable salt thereof and azacitidine or decitabine, which is a DNA methyltransferase inhibitor, in combination particularly produces an excellent anti-tumor effect and accomplished the present invention.

Specifically, the present invention relates to the following [1] to [22]:
[1] A medicament for cancer treatment comprising (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor which are administered in combination.
[2] A medicament according to [1], wherein the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor are separately contained as active ingredients in different formulations and administered at the same time or different times.
[3] A medicament according to [1], wherein the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor are contained in a single formulation.
[4] A medicament according to [1], wherein the medicament is a kit formulation comprising the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor.
[5] A method for treating cancer comprising administering (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor in combination.
[6] A treatment method according to [5], wherein the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor are separately contained as active ingredients in different formulations and administered at the same time or different times.
[7] A treatment method according to [5], wherein the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor are contained in a single formulation.
[8] A treatment method according to [5], wherein a kit formulation comprising the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor is administered.
[9] A medicament according to any one of [1] to [4], wherein the salt of the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide is p-toluenesulfonate.
[10] A treatment method according to any one of [5] to [8], wherein the salt of the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide is p-toluenesulfonate.
[11] A medicament according to any one of [1] to [4] and [9], wherein the DNA methyltransferase inhibitor is a nucleic acid derivative.
[12] A treatment method according to any one of [5] to [8] and [10], wherein the DNA methyltransferase inhibitor is a nucleic acid derivative.
[13] A medicament according to any one of [1] to [4] and [9], wherein the DNA methyltransferase inhibitor is azacitidine or decitabine.
[14] A treatment method according to any one of [5] to [8] and [10], wherein the DNA methyltransferase inhibitor is azacitidine or decitabine.
[15]A medicament according to any one of [1] to [4], [9], [11] and [13], wherein the cancer is blood cancer, brain tumor, head/neck region cancer, esophageal cancer, stomach cancer, appendix cancer, colon cancer, anus cancer, gallbladder cancer, bile duct cancer, pancreatic cancer, gastrointestinal stromal tumor, lung cancer, liver cancer, mesothelioma, thyroid gland cancer, prostate cancer, neuroendocrine tumor, melanoma, breast cancer, endometrial cancer, cervical cancer, ovarian cancer, osteosarcoma, soft tissue sarcoma, Kaposi's sarcoma, myosarcoma, renal cancer, bladder cancer or testicular cancer.
[16] A treatment method according to any one of [5] to [8], [10], [12] and [14], wherein the cancer is blood cancer, brain tumor, head/neck region cancer, esophageal cancer, stomach cancer, appendix cancer, colon cancer, anus cancer, gallbladder cancer, bile duct cancer, pancreatic cancer, gastrointestinal stromal tumor, lung cancer, liver cancer, mesothelioma, thyroid gland cancer, prostate cancer, neuroendocrine tumor, melanoma, breast cancer, endometrial cancer, cervical cancer, ovarian cancer, osteosarcoma, soft tissue sarcoma, Kaposi's sarcoma, myosarcoma, renal cancer, bladder cancer or testicular cancer.
[17]A medicament according to any one of [1] to [4], [9], [11] and [13], wherein the cancer is blood cancer.
[18] A treatment method according to any one of [5] to [8], [10], [12] and [14], wherein the cancer is blood cancer.
[19] A medicament according to [17], wherein the blood cancer is chronic lymphocytic leukemia (CLL), small lymphocytic lymphoma (SLL), high-risk CLL, non-CLL/SLL lymphoma, follicular lymphoma (FL), diffuse large B cell lymphoma (DLBCL), mantle cell lymphoma (MCL), Waldenstrom's macroglobulinemia, multiple myeloma (MM), marginal zone lymphoma, Burkitt's lymphoma, non-Burkitt high-grade B cell lymphoma, extranodal marginal zone B cell lymphoma, acute or chronic myeloid (myelocytic) leukemia, myelodysplastic syndrome or acute lymphoblastic leukemia.
[20] A treatment method according to [18], wherein the blood cancer is chronic lymphocytic leukemia (CLL), small lymphocytic lymphoma (SLL), high-risk CLL, non-CLL/SLL lymphoma, follicular lymphoma (FL), diffuse large B cell lymphoma (DLBCL), mantle cell lymphoma (MCL), Waldenstrom's macroglobulinemia, multiple myeloma (MM), marginal zone lymphoma, Burkitt's lymphoma, non-Burkitt high-grade B cell lymphoma, extranodal marginal zone B cell lymphoma, acute or chronic myeloid (myelocytic) leukemia, myelodysplastic syndrome or acute lymphoblastic leukemia.
[21] A medicament according to [17] or [19], wherein the blood cancer is cancer having wild-type TP53.
[22] A treatment method according to [18] or [20], wherein the blood cancer is cancer having wild-type TP53.

### Advantageous Effects of Invention

The present invention is useful as a method for treating cancer and/or an anti-cancer agent.

### Brief Description of Drawings

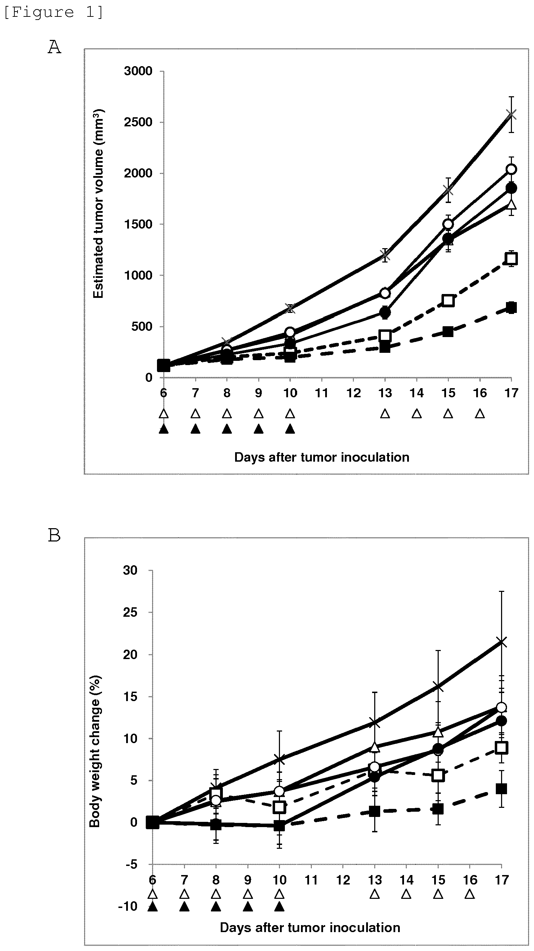

[Figure 1] Figure 1 is a diagram showing *in vivo* effects of combined use of (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide p-toluenesulfonate (Compound A) and azacitidine on tumors derived from subcutaneously transplanted cells of a human acute myeloid leukemia cell line MOLM-13 in mice (Figure 1A), and body weight change caused by combined administration thereof (Figure 1B). The symbol × depicts an untreated control group, the symbol open circle depicts 2.5 mg/kg azacitidine, the symbol filled circle depicts 4 mg/kg azacitidine, the symbol open triangle depicts 25 mg/kg Compound A, the symbol open square depicts 25 mg/kg Compound A + 2.5 mg/kg azacitidine, and the symbol filled square depicts 25 mg/kg Compound A + 4 mg/kg azacitidine. The horizontal axis shows the number of days after tumor inoculation. The vertical axis of Figure 1A shows estimated tumor volume calculated from tumor size. The vertical axis of Figure 1B shows body weight change % relative to body weight on the first day of administration. The symbol open triangle on the horizontal axis depicts the administration day of Compound A, and the filled triangle depicts the administration day of azacitidine. The error bar represents SE for the upper panel and SD for the lower panel.

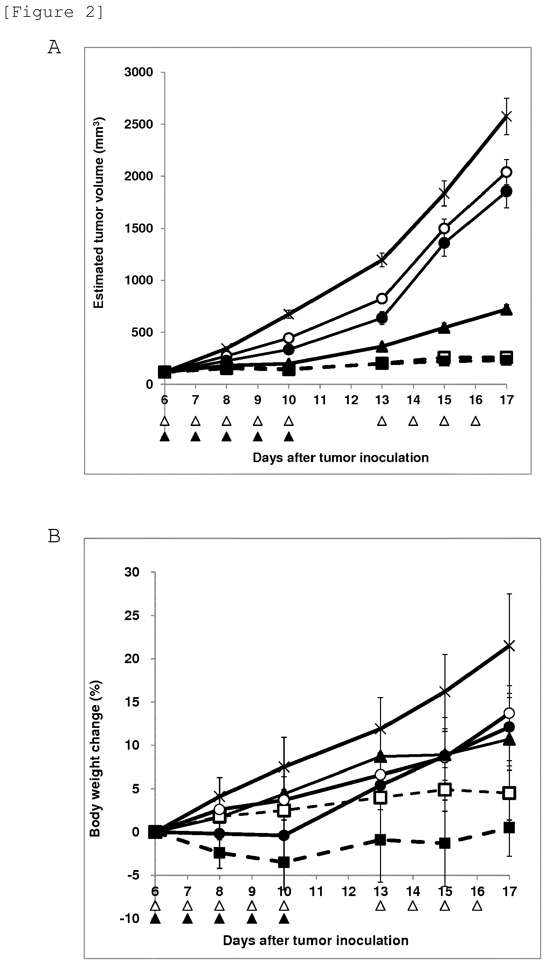

[Figure 2] Figure 2 is a diagram showing *in vivo* effects of combined use of Compound A and azacitidine on tumors derived from subcutaneously transplanted cells of a human acute myeloid leukemia cell line MOLM-13 in mice (Figure 2A), and body weight change caused by combined administration thereof (Figure 2B). The symbol × depicts an untreated control group, the symbol open circle depicts 2.5 mg/kg azacitidine, the symbol filled circle depicts 4 mg/kg azacitidine, the symbol filled triangle depicts 50 mg/kg Compound A, the symbol open square depicts 50 mg/kg Compound A + 2.5 mg/kg azacitidine, and the symbol filled square depicts 50 mg/kg Compound A + 4 mg/kg azacitidine. The horizontal axis shows the number of days after tumor inoculation. The vertical axis of Figure 2A shows estimated tumor volume calculated from tumor size. The vertical axis of Figure 2B shows body weight change % relative to body weight on the first day of administration. The symbol open triangle on the horizontal axis depicts the administration day of Compound A, and the filled triangle depicts the administration day of azacitidine. The error bar represents SE for the upper panel and SD for the lower panel.

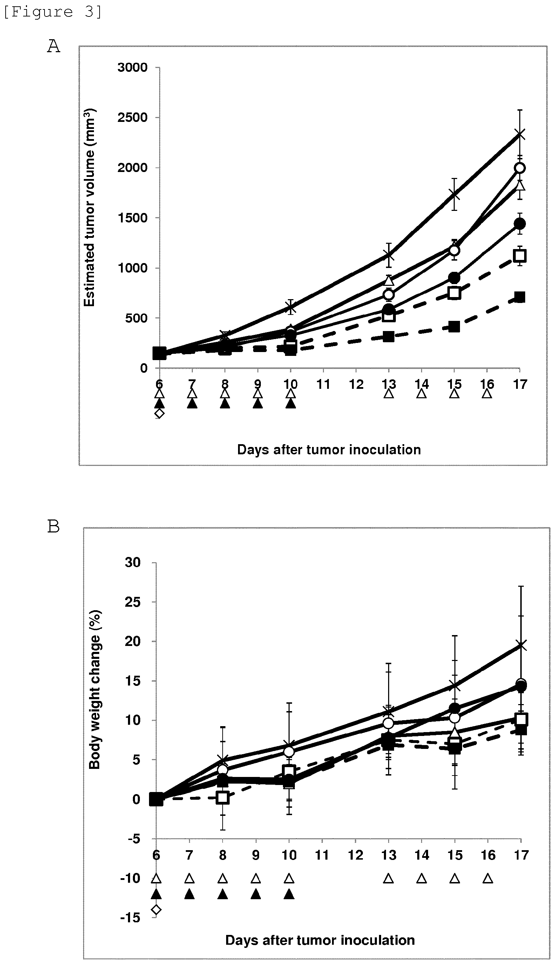

[Figure 3] Figure 3 is a diagram showing *in vivo* effects of combined use of Compound A and decitabine on tumors derived from subcutaneously transplanted cells of a human acute myeloid leukemia cell line MOLM-13 in mice (Figure 3A), and body weight change caused by combined administration thereof (Figure 3B). The symbol × depicts an untreated control group, the symbol open circle depicts 50 mg/kg decitabine, the symbol filled circle depicts 1 mg/kg decitabine, the symbol open triangle depicts 25 mg/kg Compound A, the symbol open square depicts 25 mg/kg Compound A + 50 mg/kg decitabine, and the symbol filled square depicts 25 mg/kg Compound A + 1 mg/kg decitabine. The horizontal axis shows the number of days after tumor inoculation. The vertical axis of Figure 3A shows estimated tumor volume calculated from tumor size. The vertical axis of Figure 3B shows body weight change % relative to body weight on the first day of administration. The symbol open triangle on the horizontal axis depicts the administration day of Compound A, the filled triangle depicts the administration day of 1 mg/kg decitabine, and the open rhomboid depicts the administration day of 50 mg/kg decitabine. The error bar represents SE for the upper panel and SD for the lower panel.

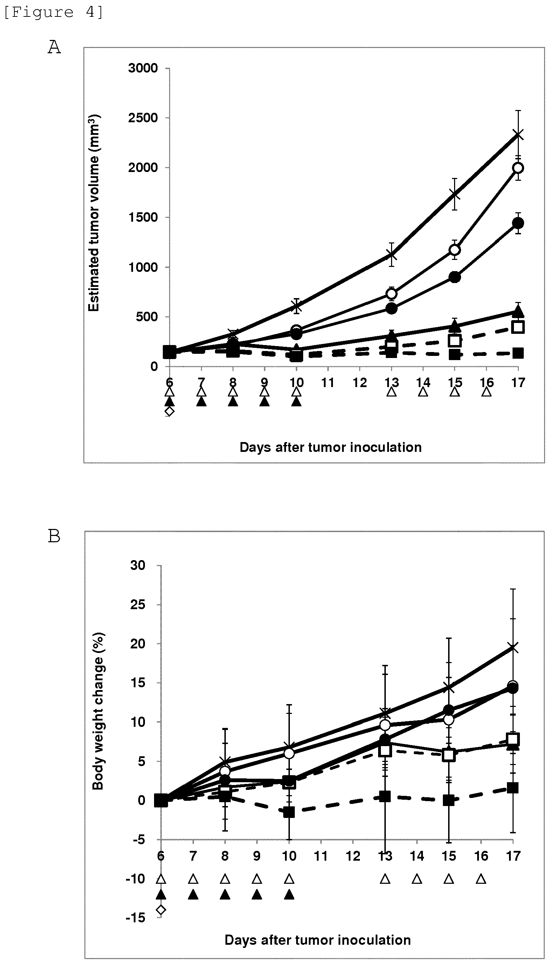

[Figure 4] Figure 4 is a diagram showing *in vivo* effects of combined use of Compound A and decitabine on tumors derived from subcutaneously transplanted cells of a human acute myeloid leukemia cell line MOLM-13 in mice (Figure 4A), and body weight change caused by combined administration thereof (Figure 4B). The symbol × depicts an untreated control group, the symbol open circle depicts 50 mg/kg decitabine, the symbol filled circle depicts 1 mg/kg decitabine, the symbol filled triangle depicts 50 mg/kg Compound A, the symbol open square depicts 50 mg/kg Compound A + 50 mg/kg decitabine, and the symbol filled square depicts 50 mg/kg Compound A +1 mg/kg decitabine. The horizontal axis shows the number of days after tumor inoculation. The vertical axis of Figure 4A shows estimated tumor volume calculated from tumor size. The vertical axis of Figure 4B shows body weight change % relative to body weight on the first day of administration. The symbol open triangle on the horizontal axis depicts the administration day of Compound A, the filled triangle depicts the administration day of 1 mg/kg decitabine, and the open rhomboid depicts the administration day of 50 mg/kg decitabine. The error bar represents SE for the upper panel and SD for the lower panel.

### Description of Embodiments

In the present invention, the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide (DS-3032) is the compound of Example 70 in WO2012/121361. This compound can be produced by a method described in WO2012/121361 (WO2012/121361 is incorporated herein by reference in its entirety).

Methylation patterns in the mammalian genome change during the initial stage when germ cells or embryos develop and during the developmental period when proteins produced by cells change. There exist 3 types of DNA methyltransferase in total: DNMT3A and DNMT3B as enzymes that establish new methylation patterns in DNA, and DNMT 1 as an enzyme that transfers the once established DNA methylation patterns to the next-generation cells in the course of replication. In the present invention, the DNA methyltransferase inhibitor may inhibit the activity of any of the enzymes described above or may inhibit the activity of all the enzymes, and is not limited by its manner of inhibition. The DNA methyltransferase inhibitor is classified mainly into nucleic acid derivatives and non-nucleic acid inhibitors. Examples of the nucleic acid derivatives include, but are not limited to, azacitidine, decitabine, zebularine, thioguanine, 5-fluoro-2'-deoxycytidine and 4'-thio-2'-deoxycytidine. Examples of the non-nucleic acid inhibitors include, but are not limited to, MG98, EGCG, psammaplins, procaine, procainamide, hydralazine, RG108, parthenolide, curcumin, mithramycin A, Nanaomycin A and MSC14778. These DNA methyltransferase inhibitors may be readily obtained as commercially available products or may be synthesized by those skilled in the art with reference to literature known in the art, etc.

The DNA methyltransferase inhibitor used in the present invention is particularly preferably azacitidine or decitabine.

In the present invention, azacitidine, 4-amino-1-β-D-ribofuranosyl-1,3,5-triazin-2(1H)-one, is also referred to as 5-azacytidine and may be referred to as Vidaza(R). Azacitidine may be readily obtained as a commercially available product.

In the present invention, decitabine, 4-amino-1-(2-deoxy-β-D-erythro-pentofuranosyl)-1,3,5-triazin-2(1H)-one, is also referred to as 5-aza-2'-deoxycytidine and may be referred to as DACOGEN(R). Decitabine may be readily obtained as a commercially available product.

In the present invention, the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide and the DNA methyltransferase inhibitor may be various pharmaceutically acceptable salts.

Examples of the salts can include: hydrohalides such as hydrochloride and hydroiodide; inorganic acid salts such as nitrate, perchlorate, sulfate and phosphate; lower alkanesulfonates such as methanesulfonate, trifluoromethanesulfonate and ethanesulfonate; arylsulfonates such as benzenesulfonate and p-toluenesulfonate; organic acid salts such as formate, acetate, malate, fumarate, succinate, citrate, tartrate, oxalate and maleate; amino acid salts such as ornithine salt, glutamate and aspartate; alkali metal salts such as sodium salt, potassium salt and lithium salt; alkaline earth metal salts such as calcium salt and magnesium salt; inorganic salts such as ammonium salt; and organic amine salts such as dibenzylamine salt, morpholine salt, phenylglycine alkyl ester salt, ethylenediamine salt, N-methylglucamine salt, diethylamine salt, triethylamine salt, cyclohexylamine salt, dicyclohexylamine salt, N,N'-dibenzylethylenediamine salt, diethanolamine salt, N-benzyl-N-(2-phenylethoxy)amine salt, piperazine salt, tetramethylammonium salt and tris(hydroxymethyl)aminomethane salt.

The salt of the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide is preferably p-toluenesulfonate.

In the present invention, the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor may each be present in a free or solvate form. The compound or the pharmaceutically acceptable salt thereof and the DNA methyltransferase inhibitor may each be present in a hydrate form, for example, by absorbing moisture in the air. The solvate is not particularly limited so long as it is pharmaceutically acceptable. Specifically, the solvate is preferably a hydrate, an ethanol solvate or the like. Moreover, an N-oxide form may be used. These solvate and N-oxide forms are also included in the scope of the present invention.

The (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor may have stereoisomers depending on their structures. The compound or the pharmaceutically acceptable salt thereof and the DNA methyltransferase inhibitor also encompass all these stereoisomers and mixtures of these stereoisomers in any ratio. The stereoisomers are as defined in 1996 IUPC, Pure and Applied Chemistry 68, 2193-2222. When the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor are each present as tautomers, these tautomers may be present in equilibrium or a certain form may be dominantly present. All these cases are included in the scope of the present invention. The tautomers refer to isomers resulting from the shift of a proton of one atom of the molecule to another atom.

The (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor may each be a "pharmaceutically acceptable prodrug compound" that is converted to the desired compound through enzymatic oxidation, reduction, hydrolysis or the like or through hydrolysis or the like induced by gastric acid or the like, due to a reaction induced by an enzyme, gastric acid or the like under physiological conditions *in vivo.*

Examples of the prodrug include compounds obtained by acylation, alkylation or phosphorylation.

Prodrugs of the compounds can be produced according to a method known in the art. Moreover, prodrugs of the compounds also include those converted to the desired compounds under physiological conditions as described in "Development of Pharmaceutical Products", vol. 7, Molecule Design, p. 163-198, Hirokawa-Shoten Ltd. (1990).

In the present invention, the terms "tumor" and "cancer" are used interchangeably. Furthermore, in the present invention, tumor, malignant tumor, cancer, malignant neoplasm, carcinoma, sarcoma, and the like may be collectively referred to as "tumor" or "cancer". Moreover, the terms "tumor" and "cancer" also include pathological conditions categorized into a premalignant stage in some cases, such as myelodysplastic syndrome.

One aspect of the present invention relates to a medicament or a treatment method comprising (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor which are administered in combination. Particularly, according to the present invention, an excellent anti-tumor effect is obtained. Hence, one aspect of the present invention relates to a medicament for cancer treatment or a method for treating cancer, comprising (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor which are administered in combination.

In the present invention, (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor "which are administered in combination" are based on the assumption that both the drugs are administered in combination.

In the present invention, the "administration in combination" of (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor means that both the drugs are incorporated into the body of a recipient over a given period. A formulation containing both the drugs in a single formulation may be administered, or the drugs may be prepared into separate formulations and separately administered. In the case of preparing separate formulations, the timing of their administration is not particularly limited. The separate formulations may be administered at the same time or may be administered at different times or on different days in a staggered manner. In the case of administering the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor at different times or on different days, the order of their administration is not particularly limited. Usually, these formulations are administered according to their respective administration methods. Therefore, these formulations may be administered in the same number of doses or may be administered in a different number of doses. Also, in the case of preparing separate formulations, the respective administration methods (administration routes) of the formulations may be the same as each other, or these formulations may be administered by different administration methods (administration routes). Both the drugs do not have to exist at the same time in the body and may be incorporated into the body over a given period (e.g., 1 month, preferably 1 week, more preferably a few days, even more preferably 1 day). One of the active ingredients may have disappeared from the body at the time of administration of the other active ingredient.

Examples of a dosage form of the medicament of the present invention include 1) administration of a single formulation comprising (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor, 2) concurrent administration through the same administration route of two formulations separately prepared from (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor, 3) administration in a staggered manner through the same administration route of two formulations separately prepared from (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro [cyclohexane-1,2'-pyrrolidine-3',3"-indole]-5'-carboxamide or a pharmaceutically acceptable salt thereof and a DNA methyltransferase inhibitor, 4) concurrent administration through different administration routes of two formulations separately prepared from (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor, and 5) administration in a staggered manner through different administrations of two formulations separately prepared from (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor.

In the present invention, the two different formulations may be in the form of a kit comprising these formulations.

A medicament according to the present invention can contain (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and/or a DNA methyltransferase inhibitor and a pharmaceutically acceptable carrier and can be administered as various injections such as intravenous injection, intramuscular injection, and subcutaneous injection or by various methods such as oral administration or percutaneous administration. A pharmaceutically acceptable carrier means a pharmacologically acceptable material that is involved in transport of a composition (e.g., an excipient, a diluent, an additive and a solvent) from a given organ to another organ.

A formulation can be prepared by selecting a suitable formulation form (e.g., an oral formulation or an injection) depending on the administration method and using various methods conventionally used for preparing a formulation. Examples of the oral formulation can include tablets, powders, granules, capsules, pills, lozenges, solutions, syrups, elixirs, emulsions and oily or aqueous suspensions. In oral administration, the free compound or a salt form may be used. An aqueous formulation can be prepared by forming an acid adduct with a pharmacologically acceptable acid or by forming an alkali metal salt such as sodium. For an injection, a stabilizer, a preservative, a dissolving aid, and the like can be used in the formulation. After filling a solution that may contain these aids and the like in a vessel, a formulation for use may be prepared as a solid formulation by lyophilization or the like. Furthermore, one dose may be filled in one vessel, or two or more doses may be filled in one vessel.

Examples of a solid formulation include tablets, powders, granules, capsules, pills and lozenges. These solid formulations may contain pharmaceutically acceptable additives together with a compound of the present invention. Examples of the additives include fillers, bulking agents, binders, disintegrating agents, dissolution promoting agents, skin wetting agents and lubricants. These additives can be selected and mixed as required to prepare a formulation.

Examples of a liquid formulation include solutions, syrups, elixirs, emulsions and suspensions. Examples of the additives include suspending agents and emulsifiers. These additives can be selected and mixed as required to prepare a formulation.

Examples of pharmaceutical materials can include, but are not limited to: amino acids such as glycine, alanine, glutamine, asparagine, arginine and lysine; antimicrobial agents; antioxidants such as ascorbic acid, sodium sulfate and sodium bisulfite; buffers such as phosphate, citrate or borate buffers, sodium bicarbonate and Tris-HCl solutions; fillers such as mannitol and glycine; chelating agents such as ethylenediaminetetraacetic acid (EDTA); complexing agents such as caffeine, polyvinylpyrrolidine, β-cyclodextrin and hydroxypropyl-β-cyclodextrin; bulking agents such as glucose, mannose and dextrin; other carbohydrates such as monosaccharides and disaccharides; coloring agents; corrigents; diluents; emulsifiers; hydrophilic polymers such as polyvinylpyrrolidine; low-molecular-weight polypeptides; salt-forming counterions; antiseptics such as benzalkonium chloride, benzoic acid, salicylic acid, thimerosal, phenethyl alcohol, methylparaben, propylparaben, chlorhexidine, sorbic acid and hydrogen peroxide; solvents such as glycerin, propylene glycol and polyethylene glycol; sugar alcohols such as mannitol and sorbitol; suspending agents; surfactants such as sorbitan ester, polysorbates such as polysorbate 20 and polysorbate 80, triton, tromethamine, lecithin and cholesterol; stability enhancers such as sucrose and sorbitol; elasticity enhancers such as sodium chloride, potassium chloride, mannitol and sorbitol; transport agents; excipients; and/or pharmaceutical aids. The amount of these pharmaceutical materials added is preferably 0.01 to 100 times, particularly, 0.1 to 10 times the weight of the drug. The recipe of a preferred pharmaceutical composition in a formulation can be appropriately determined by those skilled in the art according to an applicable disease, an applicable administration route, etc.

An excipient or a carrier in a pharmaceutical composition may be liquid or solid. Appropriate excipients or carriers may be other materials usually used in injectable water, physiological saline, artificial cerebrospinal fluid, and parenteral administration. Neutral physiological saline or physiological saline containing serum albumin may be used as a carrier. The pharmaceutical composition can contain a Tris buffer of pH 7.0 to 8.5, an acetate buffer of pH 4.0 to 5.5, or a citrate buffer of pH 3.0 to 6.2. These buffers can also contain sorbitol or other compounds.

Preferred examples of the formulation of azacitidine include, but are not limited to, injectable lyophilized formulations supplemented with D-mannitol.

Preferred examples of the formulation of decitabine include, but are not limited to, injectable lyophilized formulations containing potassium dihydrogen phosphate and sodium chloride as additives.

The medicament of the present invention can be used in cancer treatment of mammals, particularly, humans. The dose and the administration interval of the medicament of the present invention can be appropriately selected depending on the site of the disease, the patient's height, body weight, sex, or medical history, according to a physician's judgment. When the medicament of the present invention is administered to a human, the dose range is approximately 0.01 to 500 mg/kg body weight, preferably, approximately 0.1 to 100 mg/kg body weight, per day with respect to one type of active ingredient. Preferably, the active ingredient of the present invention is administered to a human once a day, or the dose is divided two to four times, and administration is repeated at an appropriate interval. Furthermore, the daily dose may exceed the above-mentioned dose at a physician's discretion, if necessary. Azacitidine is administered at 75 mg/m² (body surface area) to an adult by subcutaneous administration once a day for 7 days or by intravenous infusion over 10 minutes, followed by a 3-week drug holiday. A method of repeating administration with this operation as one cycle is a preferred example. The dose may be appropriately increased or decreased depending on the state of the patient.

Decitabine is administered at a single dose of 15 mg/m² (body surface area) to an adult by continuous intravenous infusion over 3 hours every 8 hours, and this administration is performed for 3 consecutive days. A method of repeating this administration cycle every 6 weeks, and a method of administering 20 mg/m² decitabine once a day for 5 consecutive days by continuous intravenous infusion over 1 hour or longer, followed by a 23-day drug holiday, and repeating administration with this operation as one cycle are preferred examples. The dose may be appropriately increased or decreased depending on the state of the patient.

The type of cancer to be treated is not particularly limited as long as the cancer is confirmed to be sensitive to treatment by combined use of the present invention. Examples thereof include blood cancer, brain tumor, head/neck region cancer, esophageal cancer, stomach cancer, appendix cancer, colon cancer, anus cancer, gallbladder cancer, bile duct cancer, pancreatic cancer, gastrointestinal stromal tumor, lung cancer, liver cancer, mesothelioma, thyroid gland cancer, renal cancer, prostate cancer, neuroendocrine tumor, melanoma, breast cancer, endometrial cancer, cervical cancer, ovarian cancer, osteosarcoma, soft tissue sarcoma, Kaposi's sarcoma, myosarcoma, bladder cancer and testicular cancer.

Among them, blood cancer is preferred. Examples of the blood cancer include chronic lymphocytic leukemia (CLL), small lymphocytic lymphoma (SLL), high-risk CLL, non-CLL/SLL lymphoma, follicular lymphoma (FL), diffuse large B cell lymphoma (DLBCL), mantle cell lymphoma (MCL), Waldenstrom's macroglobulinemia, multiple myeloma (MM), marginal zone lymphoma, Burkitt's lymphoma, non-Burkitt high-grade B cell lymphoma, extranodal marginal zone B cell lymphoma, acute or chronic myeloid (myelocytic) leukemia, myelodysplastic syndrome and acute lymphoblastic leukemia. Particularly preferred examples thereof include acute myeloid leukemia and myelodysplastic syndrome.

From another viewpoint, the type of cancer to be treated is preferably cancer sensitive to a MDM2 inhibitor and is more preferably cancer having wild-type TP53.

Examples of methods for confirming TP53 to be wild-type include a microarray method using a probe specific for a mutated DNA sequence (AmpliChip p53, Roche Molecular Systems, Inc., etc., http://www.ncbi.nlm.nih.gov/pubmed/21319261), PCR using a probe specific for a mutated DNA sequence (qBiomarker Somatic Mutation PCR Arrays, Qiagen N.V., etc.), a method of reading the p53 gene sequence using a Sanger sequencer (http://p53.iarc.fr/Download/TP53_DirectSequencing_IARC.p df), and a method of reading the p53 gene sequence using a next-generation sequencer (TruSeq Amplicon - Cancer Panel, Illumina http://www.illuminakk.co.jp/products/truseq_amplicon_canc er_panel.ilmn, Oncomine(R) Cancer Research Panel, Life Technologies Corp., http://www.lifetechnologies.com/jp/ja/home/clinical/precl inical-companion-diagnostic-development/oncomine-cancer-research-panel-workflow.html, etc.).

The medicament according to the present invention may be used in combination with an additional anti-tumor agent. Examples thereof include anti-tumor antibiotics, anti-tumor plant constituents, BRMs (biological response modifiers), hormones, vitamins, anti-tumor antibodies, molecular target drugs, alkylating agents, metabolic antagonists and other anti-tumor agents.

More specifically, examples of alkylating agents include: alkylating agents such as nitrogen mustard, nitrogen mustard N-oxide, bendamustine and chlorambucil; aziridine alkylating agents such as carboquone and thiotepa; epoxide alkylating agents such as dibromomannitol and dibromodulcitol; nitrosourea alkylating agents such as carmustine, lomustine, semustine, nimustine hydrochloride, streptozocin, chlorozotocin and ranimustine; and busulfan, improsulfan tosylate, temozolomide and dacarbazine.

Various examples of metabolic antagonists include: purine metabolic antagonists such as 6-mercaptopurine, 6-thioguanine and thioinosine; pyrimidine metabolic antagonists such as fluorouracil, tegafur, tegafur-uracil, carmofur, doxifluridine, broxuridine, cytarabine and enocitabine; and folic acid metabolic antagonists such as methotrexate and trimetrexate.

Examples of anti-tumor antibiotics include: mitomycin C, bleomycin, peplomycin, daunorubicin, aclarubicin, doxorubicin, idarubicin, pirarubicin, THP-adriamycin, 4'-epidoxorubicin and epirubicin; and chromomycin A3 and actinomycin D.

Examples of anti-tumor plant constituents and their derivatives include: vinca alkaloids such as vindesine, vincristine and vinblastine; taxanes such as paclitaxel, docetaxel and cabazitaxel; and epipodophyllotoxins such as etoposide and teniposide.

Examples of BRMs include tumor necrosis factors and indomethacin.

Examples of hormones include hydrocortisone, dexamethasone, methylprednisolone, prednisolone, prasterone, betamethasone, triamcinolone, oxymetholone, nandrolone, metenolone, fosfestrol, ethinylestradiol, chlormadinone, mepitiostane and medroxyprogesterone.

Examples of vitamins include vitamin C and vitamin A.

Examples of anti-tumor antibodies and molecular target drugs include trastuzumab, rituximab, cetuximab, nimotuzumab, denosumab, bevacizumab, infliximab, ipilimumab, nivolumab, pembrolizumab, avelumab, pidilizumab, atezolizumab, ramucirumab, imatinib mesylate, dasatinib, gefitinib, erlotinib, osimertinib, sunitinib, lapatinib, dabrafenib, trametinib, cobimetinib, pazopanib, palbociclib, panobinostat, sorafenib, crizotinib, vemurafenib, quizartinib, bortezomib, carfilzomib, ixazomib, midosutaurin and gilteritinib.

Examples of other anti-tumor agents include cisplatin, carboplatin, oxaliplatin, tamoxifen, letrozole, anastrozole, exemestane, toremifene citrate, fulvestrant, bicalutamide, flutamide, mitotane, leuprorelin, goserelin acetate, camptothecin, ifosfamide, cyclophosphamide, melphalan, L-asparaginase, aceglatone, sizofiran, picibanil, procarbazine, pipobroman, neocarzinostatin, hydroxyurea, ubenimex, thalidomide, lenalidomide, pomalidomide, eribulin, tretinoin and krestin.

### Examples

Hereinafter, the present invention will be specifically explained with reference to the Examples given below. However, the present invention is not limited to these examples, and they should not be construed in any limitative way.

### (Test Example 1 Study on in vivo effect of combined use of Compound A and azacitidine)

Human acute myeloid leukemia cell line MOLM-13 cells were suspended to 5 × 10⁷ cells/mL using phosphate-buffered saline. 0.1 mL of the prepared cell suspension was subcutaneously transplanted to each NOD-SCID mouse (male, 6 weeks old). On 6 days after the tumor inoculation, after confirmation that the average tumor volume exceeded 100 mm³, the mice were grouped on the basis of their tumor volume values. 25 mg/kg or 50 mg/kg Compound A was orally administered by forced administration to the mice. 2.5 mg/kg or 4 mg/kg azacitidine was intravenously administered to the tails of the mice. For a combined use group, 25 mg/kg or 50 mg/kg Compound A and 2.5 mg/kg or 4 mg/kg azacitidine were administered sequentially. The administration of Compound A was performed once a day for 5 consecutive days (6 to 10 days after the tumor inoculation) from the date of grouping (6 days after the tumor inoculation), and after a 2-day drug holiday, performed once a day for 4 consecutive days (13 to 16 days after the tumor inoculation). The administration of azacitidine was performed once a day for 5 consecutive days (6 to 10 days after the tumor inoculation) from the date of grouping (6 days after the tumor inoculation). The major axis (mm) and minor axis (mm) of tumor were measured over time using an electronic digital caliper. Tumor growth inhibition % (TGI%) on the date of assessment (17 days after the tumor inoculation) calculated according to calculation formula (4) shown below was used in evaluation. Also, the body weights were measured over time using an automatic balance for small animals, and body weight change % was calculated according to calculation formula (5) shown below to study the influence of drug administration on the body weights. In addition, the results of the last body weight measurement were used in dose calculation.$TGI \left(%\right) = \left(1-A/B\right) \times 100$
A: Average tumor volume of the compound-administered group on the date of assessment (*)
B: Average tumor volume of the untreated control group on the date of assessment (*)
*: The tumor volume was calculated according to 1/2 × [Major axis of tumor] × [Minor axis of tumor] × [Minor axis of tumor].
$Body weight change \left(%\right) = Average body weight change % of the individuals$ $Body weight change % of each individual = \left(1-BWn/BWs\right) \times 100$
BWn: Body weight on day n
BWs: Body weight on the start day of administration

The results are shown in Figures 1 and 2 and Tables 1 to 3.

**[Table 1]**

| Group | TGI (%) |
|---|---|
| Compound A 25 mg/kg | 34 |
| Compound A 50 mg/kg | 72 |
| Compound A 25 mg/kg + Azacitidine 2.5 mg/kg | 55 |
| Compound A 50 mg/kg + Azacitidine 2.5 mg/kg | 90 |
| Compound A 25 mg/kg + Azacitidine 4 mg/kg | 73 |
| Compound A 50 mg/kg + Azacitidine 4 mg/kg | 91 |
| Azacitidine 2.5 mg/kg | 21 |
| Azacitidine 4 mg/kg | 28 |

Estimated tumor volume (mm³)

**[Table 2]**

| Group | | Number of days after tumor inoculation | | | | | |
|---|---|---|---|---|---|---|---|
| | | 6 | 8 | 10 | 13 | 15 | 17 |
| Untreated | average | 116 | 345 | 674 | 1196 | 1835 | 2575 |
| | SE | 5 | 12 | 38 | 65 | 119 | 176 |
| Compound A 25 mg/kg | average | 118 | 269 | 418 | 834 | 1346 | 1697 |
| | SE | 5 | 20 | 30 | 38 | 94 | 111 |
| Compound A 50 mg/kg | average | 116 | 180 | 197 | 364 | 545 | 721 |
| | SE | 6 | 14 | 15 | 23 | 43 | 44 |
| Compound A 25 mg/kg + Azacitidine 2.5 mg/kg | average | 118 | 202 | 241 | 409 | 751 | 1163 |
| | SE | 5 | 8 | 18 | 36 | 57 | 78 |
| Compound A 50 mg/kg + Azacitidine 2.5 mg/kg | average | 117 | 152 | 141 | 200 | 257 | 258 |
| | SE | 6 | 4 | 7 | 12 | 15 | 22 |
| Compound A 25 mg/kg + Azacitidine 4 mg/kg | average | 117 | 180 | 200 | 295 | 450 | 684 |
| | SE | 5 | 11 | 13 | 23 | 43 | 57 |
| Compound A 50 mg/kg + Azacitidine 4 mg/kg | average | 118 | 164 | 146 | 198 | 221 | 230 |
| | SE | 5 | 8 | 10 | 18 | 19 | 18 |
| Azacitidine 2.5 mg/kg | average | 117 | 272 | 444 | 823 | 1500 | 2039 |
| | SE | 5 | 8 | 26 | 33 | 89 | 122 |
| Azacitidine 4 mg/kg | average | 115 | 226 | 334 | 637 | 1359 | 1855 |
| | SE | 7 | 28 | 36 | 61 | 128 | 157 |

Body weight change (%)

**[Table 3]**

| Group | | Number of days after tumor inoculation | | | | | |
|---|---|---|---|---|---|---|---|
| | | 6 | 8 | 10 | 13 | 15 | 17 |
| Untreated | average | 0.0 | 4.1 | 7.5 | 11.9 | 16.2 | 21.5 |
| | SD | 0.0 | 2.2 | 3.4 | 3.6 | 4.3 | 6.0 |
| Compound A 25 mg/kg | average | 0.0 | 2.5 | 3.7 | 9.0 | 10.8 | 13.8 |
| | SD | 0.0 | 2.7 | 2.3 | 2.9 | 3.6 | 3.7 |
| Compound A 50 mg/kg | average | 0.0 | 1.7 | 4.4 | 8.7 | 8.9 | 10.7 |
| | SD | 0.0 | 2.6 | 2.0 | 3.2 | 4.3 | 3.6 |
| Compound A 25 mg/kg + Azacitidine 2.5 mg/kg | average | 0.0 | 3.4 | 1.8 | 6.2 | 5.6 | 8.9 |
| | SD | 0.0 | 2.3 | 3.3 | 3.0 | 3.0 | 1.8 |
| Compound A 50 mg/kg + Azacitidine 2.5 mg/kg | average | 0.0 | 1.8 | 2.5 | 4.0 | 4.9 | 4.5 |
| | SD | 0.0 | 2.2 | 1.1 | 1.4 | 2.5 | 3.1 |
| Compound A 25 mg/kg + Azacitidine 4 mg/kg | average | 0.0 | -0.3 | -0.4 | 1.3 | 1.6 | 4.0 |
| | SD | 0.0 | 2.2 | 2.2 | 2.4 | 1.9 | 2.2 |
| Compound A 50 mg/kg + Azacitidine 4 mg/kg | average | 0.0 | -2.4 | -3.5 | -0.9 | -1.3 | 0.5 |
| | SD | 0.0 | 1.8 | 3.2 | 4.9 | 5.0 | 3.3 |
| Azacitidine 2.5 mg/kg | average | 0.0 | 2.6 | 3.7 | 6.6 | 8.6 | 13.7 |
| | SD | 0.0 | 1.6 | 1.2 | 2.5 | 0.6 | 3.2 |
| Azacitidine 4 mg/kg | average | 0.0 | -0.2 | -0.4 | 5.4 | 8.8 | 12.1 |
| | SD | 0.0 | 1.9 | 2.7 | 1.3 | 2.8 | 3.9 |

### (Test Example 2 Study on in vivo effect of combined use of Compound A and decitabine)

Human acute myeloid leukemia cell line MOLM-13 cells were suspended to 5 × 10⁷ cells/mL using phosphate-buffered saline. 0.1 mL of the prepared cell suspension was subcutaneously transplanted to each NOD-SCID mouse (male, 6 weeks old). On 6 days after the tumor inoculation, after confirmation that the average tumor volume exceeded 100 mm³, the mice were grouped on the basis of their tumor volume values. 25 mg/kg or 50 mg/kg Compound A was orally administered by forced administration to the mice. 1 mg/kg or 50 mg/kg decitabine was intravenously administered to the tails of the mice. For a combined use group, 25 mg/kg or 50 mg/kg Compound A and 1 mg/kg or 50 mg/kg decitabine were administered sequentially. The administration of Compound A was performed once a day for 5 consecutive days (6 to 10 days after the tumor inoculation) from the date of grouping (6 days after the tumor inoculation), and after a 2-day drug holiday, performed once a day for 4 consecutive days (13 to 16 days after the tumor inoculation). The administration of decitabine to the 50 mg/kg group was performed at the date of grouping (6 days after the tumor inoculation), and the administration thereof to the 1 mg/kg group was performed once a day for 5 consecutive days (6 to 10 days after the tumor inoculation) from the date of grouping (6 days after the tumor inoculation). The major axis (mm) and minor axis (mm) of tumor were measured over time using an electronic digital caliper. Tumor growth inhibition % (TGI%) on the date of assessment (17 days after the tumor inoculation) calculated according to calculation formula (4) shown below was used in evaluation. Also, the body weights were measured over time using an automatic balance for small animals, and body weight change % was calculated according to calculation formula (5) shown below to study the influence of drug administration on the body weights. In addition, the results of the last body weight measurement were used in dose calculation.$TGI \left(%\right) = \left(1-A/B\right) \times 100$
A: Average tumor volume of the compound-administered group on the date of assessment (*)
B: Average tumor volume of the untreated control group on the date of assessment (*)
*: The tumor volume was calculated according to 1/2 × [Major axis of tumor] × [Minor axis of tumor] × [Minor axis of tumor].
$Body weight change \left(%\right) = Average body weight change % of the individuals$ $Body weight change % of each individual = \left(1-BWn/BWs\right) \times 100$
BWn: Body weight on day n
BWs: Body weight on the start day of administration

The results are shown in Figures 3 and 4 and Tables 4 to 6.

**[Table 4]**

| Group | TGI (%) |
|---|---|
| Compound A 25 mg/kg | 22 |
| Compound A 50 mg/kg | 76 |
| Compound A 25 mg/kg + Decitabine 50 mg/kg | 52 |
| Compound A 50 mg/kg + Decitabine 50 mg/kg | 83 |
| Compound A 25 mg/kg + Decitabine 1 mg/kg | 70 |
| Compound A 50 mg/kg + Decitabine 1 mg/kg | 94 |
| Decitabine 50 mg/kg | 14 |
| Decitabine 1 mg/kg | 38 |

Estimated tumor volume (mm³)

**[Table 5]**

| Group | | Number of days after tumor inoculation | | | | | |
|---|---|---|---|---|---|---|---|
| | | 6 | 8 | 10 | 13 | 15 | 17 |
| Untreated | average | 143 | 327 | 609 | 1127 | 1733 | 2332 |
| | SE | 10 | 35 | 74 | 119 | 159 | 242 |
| Compound A 25 mg/kg | average | 147 | 257 | 388 | 876 | 1213 | 1825 |
| | SE | 11 | 17 | 19 | 48 | 70 | 141 |
| Compound A 50 mg/kg | average | 143 | 227 | 170 | 310 | 407 | 554 |
| | SE | 9 | 31 | 19 | 58 | 79 | 91 |
| Compound A 25 mg/kg + Decitabine 50 mg/kg | average | 148 | 200 | 216 | 525 | 750 | 1120 |
| | SE | 11 | 7 | 12 | 37 | 66 | 97 |
| Compound A 50 mg/kg + Decitabine 50 mg/kg | average | 146 | 159 | 120 | 200 | 260 | 397 |
| | SE | 10 | 11 | 4 | 15 | 26 | 57 |
| Compound A 25 mg/kg + Decitabine 1 mg/kg | average | 143 | 182 | 179 | 315 | 415 | 708 |
| | SE | 10 | 9 | 12 | 23 | 33 | 52 |
| Compound A 50 mg/kg + Decitabine 1 mg/kg | average | 148 | 151 | 101 | 141 | 119 | 136 |
| | SE | 12 | 12 | 6 | 14 | 12 | 14 |
| Decitabine 50 mg/kg | average | 145 | 206 | 365 | 732 | 1175 | 1997 |
| | SE | 10 | 22 | 49 | 68 | 96 | 124 |
| Decitabine 1 mg/kg | average | 144 | 228 | 327 | 585 | 901 | 1441 |
| | SE | 11 | 18 | 25 | 41 | 54 | 105 |

Body weight change (%)

**[Table 6]**

| Group | | Number of days after tumor inoculation | | | | | |
|---|---|---|---|---|---|---|---|
| | | 6 | 8 | 10 | 13 | 15 | 17 |
| Untreated | average | 0.0 | 4.9 | 6.8 | 11.1 | 14.4 | 19.5 |
| | SD | 0.0 | 4.3 | 4.3 | 6.1 | 6.3 | 7.5 |
| Compound A 25 mg/kg | average | 0.0 | 2.3 | 2.0 | 8.0 | 8.5 | 10.3 |
| | SD | 0.0 | 1.8 | 3.0 | 2.7 | 4.2 | 3.2 |
| Compound A 50 mg/kg | average | 0.0 | 1.7 | 2.4 | 7.4 | 6.2 | 7.2 |
| | SD | 0.0 | 2.5 | 4.1 | 3.2 | 3.6 | 3.7 |
| Compound A 25 mg/kg + Decitabine 50 mg/kg | average | 0.0 | 0.2 | 3.5 | 7.5 | 7.0 | 10.1 |
| | SD | 0.0 | 2.2 | 1.8 | 4.4 | 5.7 | 4.5 |
| Compound A 50 mg/kg + Decitabine 50 mg/kg | average | 0.0 | 1.1 | 2.3 | 6.4 | 5.8 | 7.8 |
| | SD | 0.0 | 1.9 | 1.7 | 1.8 | 3.5 | 3.2 |
| Compound A 25 mg/kg +Decitabine 1 mg/kg | average | 0.0 | 2.2 | 2.2 | 6.9 | 6.4 | 8.8 |
| | SD | 0.0 | 2.3 | 2.2 | 1.1 | 1.9 | 2.4 |
| Compound A 50 mg/kg + Decitabine 1 mg/kg | average | 0.0 | 0.5 | -1.5 | 0.5 | 0.0 | 1.6 |
| | SD | 0.0 | 2.9 | 3.5 | 7.3 | 5.4 | 5.7 |
| Decitabine 50 mg/kg | average | 0.0 | 3.7 | 6.0 | 9.6 | 10.3 | 14.6 |
| | SD | 0.0 | 3.6 | 6.2 | 6.5 | 7.3 | 8.6 |
| Decitabine 1 mg/kg | average | 0.0 | 2.6 | 2.5 | 7.8 | 11.5 | 14.3 |
| | SD | 0.0 | 6.5 | 4.4 | 3.9 | 4.2 | 5.5 |

## Claims

1. A medicament for cancer treatment comprising (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor which are administered in combination.

2. A medicament according to claim 1, wherein the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor are separately contained as active ingredients in different formulations and administered at the same time or different times.

3. A medicament according to claim 1, wherein the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor are contained in a single formulation.

4. A medicament according to claim 1, wherein the medicament is a kit formulation comprising the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor.

5. A method for treating cancer comprising administering (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or a pharmaceutically acceptable salt thereof and a DNA methyltransferase inhibitor in combination.

6. A treatment method according to claim 5, wherein the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor are separately contained as active ingredients in different formulations and administered at the same time or different times.

7. A treatment method according to claim 5, wherein the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor are contained in a single formulation.

8. A treatment method according to claim 5, wherein a kit formulation comprising the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide or the pharmaceutically acceptable salt thereof and the DNA methyltransferase inhibitor is administered.

9. A medicament according to any one of claims 1 to 4, wherein the salt of the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide is p-toluenesulfonate.

10. A treatment method according to any one of claims 5 to 8, wherein the salt of the (3'R,4'S,5'R)-N-[(3R,6S)-6-carbamoyltetrahydro-2H-pyran-3-yl]-6"-chloro-4'-(2-chloro-3-fluoropyridin-4-yl)-4,4-dimethyl-2"-oxo-1",2"-dihydrodispiro[cyclohexane-1,2'-pyrrolidine-3',3"-indole]-5'-carboxamide is p-toluenesulfonate.

11. A medicament according to any one of claims 1 to 4 and 9, wherein the DNA methyltransferase inhibitor is a nucleic acid derivative.

12. A treatment method according to any one of claims 5 to 8 and 10, wherein the DNA methyltransferase inhibitor is a nucleic acid derivative.

13. A medicament according to any one of claims 1 to 4 and 9, wherein the DNA methyltransferase inhibitor is azacitidine or decitabine.

14. A treatment method according to any one of claims 5 to 8 and 10, wherein the DNA methyltransferase inhibitor is azacitidine or decitabine.

15. A medicament according to any one of claims 1 to 4, 9, 11 and 13, wherein the cancer is blood cancer, brain tumor, head/neck region cancer, esophageal cancer, stomach cancer, appendix cancer, colon cancer, anus cancer, gallbladder cancer, bile duct cancer, pancreatic cancer, gastrointestinal stromal tumor, lung cancer, liver cancer, mesothelioma, thyroid gland cancer, prostate cancer, neuroendocrine tumor, melanoma, breast cancer, endometrial cancer, cervical cancer, ovarian cancer, osteosarcoma, soft tissue sarcoma, Kaposi's sarcoma, myosarcoma, renal cancer, bladder cancer or testicular cancer.

16. A treatment method according to any one of claims 5 to 8, 10, 12 and 14, wherein the cancer is blood cancer, brain tumor, head/neck region cancer, esophageal cancer, stomach cancer, appendix cancer, colon cancer, anus cancer, gallbladder cancer, bile duct cancer, pancreatic cancer, gastrointestinal stromal tumor, lung cancer, liver cancer, mesothelioma, thyroid gland cancer, prostate cancer, neuroendocrine tumor, melanoma, breast cancer, endometrial cancer, cervical cancer, ovarian cancer, osteosarcoma, soft tissue sarcoma, Kaposi's sarcoma, myosarcoma, renal cancer, bladder cancer or testicular cancer.

17. A medicament according to any one of claims 1 to 4, 9, 11 and 13, wherein the cancer is blood cancer.

18. A treatment method according to any one of claims 5 to 8, 10, 12 and 14, wherein the cancer is blood cancer.

19. A medicament according to claim 17, wherein the blood cancer is chronic lymphocytic leukemia (CLL), small lymphocytic lymphoma (SLL), high-risk CLL, non-CLL/SLL lymphoma, follicular lymphoma (FL), diffuse large B cell lymphoma (DLBCL), mantle cell lymphoma (MCL), Waldenstrom's macroglobulinemia, multiple myeloma (MM), marginal zone lymphoma, Burkitt's lymphoma, non-Burkitt high-grade B cell lymphoma, extranodal marginal zone B cell lymphoma, acute or chronic myeloid (myelocytic) leukemia, myelodysplastic syndrome or acute lymphoblastic leukemia.

20. A treatment method according to claim 18, wherein the blood cancer is chronic lymphocytic leukemia (CLL), small lymphocytic lymphoma (SLL), high-risk CLL, non-CLL/SLL lymphoma, follicular lymphoma (FL), diffuse large B cell lymphoma (DLBCL), mantle cell lymphoma (MCL), Waldenstrom's macroglobulinemia, multiple myeloma (MM), marginal zone lymphoma, Burkitt's lymphoma, non-Burkitt high-grade B cell lymphoma, extranodal marginal zone B cell lymphoma, acute or chronic myeloid (myelocytic) leukemia, myelodysplastic syndrome or acute lymphoblastic leukemia.

21. A medicament according to claim 17 or 19, wherein the blood cancer is cancer having wild-type TP53.

22. A treatment method according to claim 18 or 20, wherein the blood cancer is cancer having wild-type TP53.
